# EUROPÄISCHE PATENTSCHRIFT

(11) **EP 1 691 894 B1**
(45) Veröffentlichungstag und Bekanntmachung des Hinweises auf die Patenterteilung: **22.02.2012**
(21) Anmeldenummer: 04790132.7
(22) Anmeldetag: 05.10.2004
(51) Int. Cl.: A62B 18/10, A62B 9/00, A61M 16/06

(54) **ATEMMASKE**
RESPIRATORY MASK
MASQUE RESPIRATOIRE

(30) Priorität: 28.11.2003 DE 10355752
(43) Veröffentlichungstag der Anmeldung: 23.08.2006
(73) Patentinhaber: Drägerwerk AG, 23542 Lübeck (DE)
(72) Erfinder: HANSMANN, Hans-Ulrich, 23858 Barnitz (DE); KULLIK, Götz, 23568 Lübeck (DE); MEYER, Jörg-Uwe, 23909 Ratzeburg (DE)
(74) Vertreter: UEXKÜLL & STOLBERG
(86) Internationale Anmeldenummer: PCT/EP2004/011125
(87) Internationale Veröffentlichungsnummer: WO 2005/061054

(56) Entgegenhaltungen:
- WO-A-96/35911
- GB-A- 504 232
- GB-A- 825 659
- GB-A- 2 264 060
- US-A- 440 713
- US-A- 3 814 094
- US-A- 4 904 394
- US-A- 5 595 173
- US-A1- 2003 005 934
- US-A1- 2003 164 170

## Beschreibung

Die Erfindung betrifft eine Atemmaske mit den Merkmalen des Oberbegriffs des Patentanspruchs 1.

Eine Atemmaske der genannte Art ist aus der US 4,971,051 bekannt. Sie besteht aus einem Maskenkörper mit einer Einatemöffnung und einer Ausatemöffnung, und ist mittels einer Bänderung am Gesicht eines Maskenträgers befestigt. Die Abdichtung zwischen Gesicht und Maskenkörper erfolgt, über einen am Umfang des Maskenkörpers verlaufenden Dichtrand. Mit einer Druckgasquelle, die an die Einatemöffnung angeschlossen ist, wird ein kontiriuierlicher Atemgasfluss bei konstantem Überdruck im Maskeninnenraum erzeugt, um eine CPAP-Beatmung . (Continous Positive Airway Pressure) durchführen zu können.

Nachteilig bei der bekannten Atemmaske ist, dass der kontinuierliche Gasaustritt aus der Ausatemöffnung mit einem nicht zu vemachlässigenderi Geräuschpegel verbünden ist, der insbesondere beim Einsatz der Atemmaske im häuslichen Bereich nicht toleriert werden kann. Ein derartiger Einsatz liegt zum Beispiel bei oder Behandlung einer Schlafapnoe vor.

Eine weitere Atemmaske ist aus GB 825,659 A bekannt, die eine Ausatemeinrichtung aufweist, welche eine erste Lage mit einer Mehrzahl von kreisförmigen Öffnungen hat, wobei jede Öffnung von einem elastischen Membranelement bedeckt wird. Jedes Membranelement ist nur auf einer Seite der zugehörigen Öffnung befestigt, so dass es zur Freigabe der zugehörigen Öffnung wegklappen kann. Die insgesamt realisierbare Ausströmfläche bleibt als Summe der Kreisöffnungsflächen jedoch begrenzt.

Der Erfindung liegt die Aufgabe zugrunde, eine Atemmaske der genannten Art derart zu verbessern, dass der Gasaustritt an der Ausatemöffnung ohne nennenswerte Geräuschbelästigung möglich ist.

Die Lösung der Aufgabe erfolgt mit den Merkmalen des Patentanspruchs 1.

Der Vorteil der Erfindung besteht im Wesentlichen darin, dass durch eine Vielzahl von teilweise überlappend, lamellenartig am Maskenkörper angeordneten, von Ausatemstrom durchströmbaren Membranalementen eine große Fläche für den Abfluss des Ausatemgases und des für eine CPAP-Beatmüng erforderlichen Basisgasstroms bereitgestellt wind, so dass ein Gasfluss mit geringer Strömungsgeschwindigkeit möglich ist.

Über die Geometrie der Membranelerriente und das Zusammenspiel von Eigenelastizität und Porosität lässt sich ein bestimmter pneumatischer Widerstand einstellen, aus dem sich ein definierter Basisdruck im Maskeninnenraum für die CPAP-Beatrnürig ergibt. Durch Veränderung der physikalischen Eigenschaften der Membranelemente lässt sich für jeden CPAP-Druck eine individuelle Maske herstellen, die über die Einatemöffnung an eine unspezifische Hochdruckquelle angeschlossen werden kann, wobei das Überschussgas nach außen über die Membranelemente abfliessen kann.

Die erfindungsgemäß angegebene Maske lässt sich aus flachem; leichtem Material mit wenig Verpackung herstellen und besitzt daher gute Trageeigenschaften. Die Membranelemente können dabei als streifenförmige Bauteile zu einer Tuchkonstruktion zusammengefügt sein, wobei die Steifigkeit durch integrierte Titan-Nickelfäden beeinflusst werden kann.

Ein zwischen dem Maskenkörper und dem Gesicht des Maskentfägers angeordneter Dichtrand besteht aus weichem, komfortablen Elastomermaterial, welches sich gut der Gesichtsform anpasst. Sofern der Maskenkörper aus nachgiebigem Material besteht, kann der Dichtrand durch einen steifen, aber formbaren Rahmen gestützt werden. Neben einfachen Metallrahmen ist eine Konstruktion auf der Basis von "Formgedächtnislegierungen" vorteilhaft, die bei tiefen Temperaturen, zum Beispiel bei kurzzeitiger Lagerung im Gefrierfach, eine plastische Verformung ermöglichen.

Vorteilhafte Ausgestaltungen der Erfindung ergeben sich aus den Unteränsprüche.

Die Membranelemente sind lamellenartig, teilweise. überlappend und vom Ausatemgasstrom durchströmbar am Maskenkörper anbeordnet Beim Durchtritt des Ausatsmgässfröms werden die Membranelemente teilweise oder auch vollständig aufgeklappt. Über Anzahl und Geometrie. der Membranelemente sowie deren Federsteifigkeit lässt sich der Basisdruck im Maskeninnenraum beeinflussen.
in vorteilhafter Weise sind die Membranefemente in Form von einseitig befestigten Biegebalken ausgeführt, wobei die Einspannstellen im Überlappungsbereich der Membranelemente liegen. Die Membranelemente können hierbei auf ein poröses Trägermaterial aufgeklebt sein und werden durch den durch das Trägermaterial hindurchströmenden Gasstrom aufgeklappt.

Das Membranmaterial besteht vorteilhaft aus einem textilen -Werkstoff oder aus einem Elastomer, wobei das Material stückweise oder auch vollständig gasper meabel sein kann.

Um die Federsteifigkeit des Materials zu beeinflussen, kann eine Materlafkomponente integriert sein, die ihre mechanische Geometrie, ähnlich wie elektrorheologische Flüssigkeiten, infolge elektrischer Signale direkt verändert. Die Membranelemente können aber auch vollständig aus der Materialkomponente bestehen.

Es besteht vorteilhaft auch die Möglichkeit, als Membranmaterial eine PVDF-Fotie zu verwenden, deren Steifigkeit durch elektrische Felder verändert werden kann. Über die elektrische Beeinflussung der Federsteifigkeit lässt sich so eine elektrische Modulation des Atemgasflusses vornehmen. Hierdurch ist die erfindungsgemäße Atemmaske auch für Beatmungsformen mit Unterschiedlichen CPAP-Druckstufen und für maschinelle- oder Spöntanatmungsunterstützung geeignet.

Ein Ausführungsbeispiel der Ereindung ist in der Figur gezeigt und im Folgenden näher erläutert.

Es zeigen:
- Figur 1: eine erste Atemmaske nach der Erfindung im Längsschnitt,
- Figur 2: die Einzelheit A nach der Figur 1 ohne Gasdurchströmung,
- Figur 3: die Einzelheit A nach der Figur 1 mit Gäsdurchströmung,
- Figur 4: eine zweite Atemmaske im Längsschnitte, die hierin nicht beansprucht ist,
- Figur 5 ,: die Einzelheit B nach der Figur 4,
- Figur 6: die Einzelheit B nach der Figur 4 mit verengten Strömungskanälen,
- Figur 7: die Einzelheit B mit Membranfolien,
- Figur 8: die Einzelbeit B mit Membranfolien, die mit einer Potentialspannungsquelle verbunden sind,

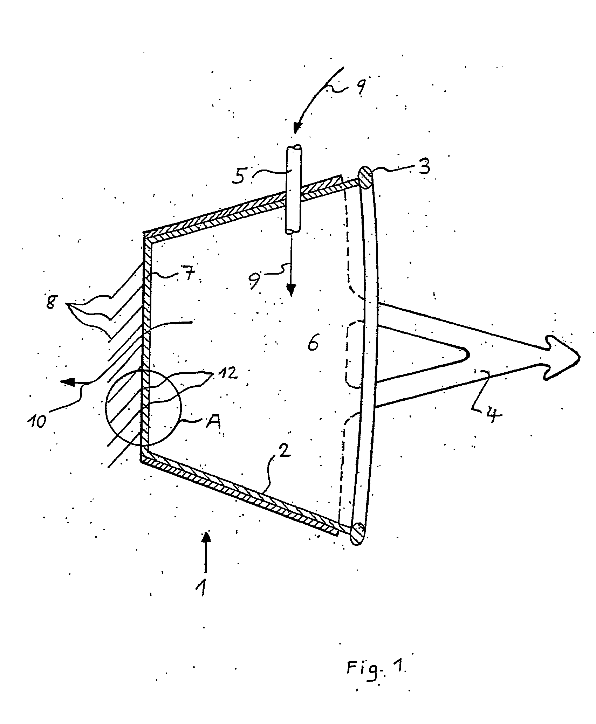

Figur 1 zeigt schematisch eine-erste Atemmaske 1 nach der Erfindung im Längsschnitt. An einem Maskenkörper 2 befindet sich ein umlaufender Dichtrand 3, der am Gesicht eines-in der Figur 1 nicht näher dargestellten Maskenträgers anliegt. Die erste Atemmaske 1 wird mit einer in der Figur 1 nur stückweise dargestellten Bänderung 4 am Kopf des Maskenträgers fixiert. Das Atemgas gelangt über eine Einatemöffnung 5 in einen Maskeninnenraum 6. An der Vorderseite des Maskenkorpers 2 befindet sich ein gasdruchlässiges Trägermaterial 7, an dem streifenförmige, lamellenartig angeordnete Membranelemente 8 in Form von Biegebalken an Einspannstellen 12 befestigt sind.

Figur 1 veranschaulicht die Membranelemente 8 im gasdurchströmten Zustands der ersten Atemmaske 1, bei dem die Membranelemente 8 durch den Gasstrom von dem Trägermaterial 7 angehoben werden. Die Durchströmungsrichtung ist durch Pfeile 9, 10 Veranschaulicht.

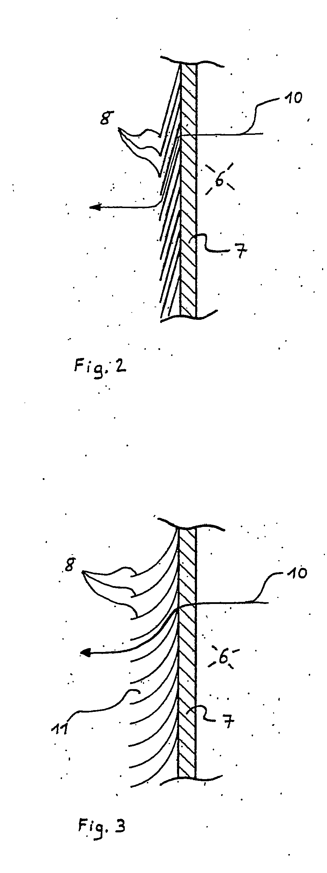

Figur 2 veranschaulicht die Einzelheit A nach der Figur 1 für eine nicht vom Gas durchströmte erste Atemmaske 1. Die Membranelemente 8 liegen hierbei überlappend aufeinander, so dass das Trägermaterial 7 durch die Membranelemente 8 abgedeckt ist und kein Gas aus der Umgebung in den Maskeninnenraum 6 gelangen kann. Gleiche Komponenten sind mit gleichen Bezugsziffern der Figur 1 versehen.

Figur 3 veränschaulicht die Einzelheit A nach der Figur 1 für eine Gasdurchströmung des Trägermaterial 7 längst des Pfeils 10. Die Membranelemente 8 werden hierbei als Biegebalken verformt, so dass sich zwischen benachbarten Membranelementen 8 Strömungskartäle 11 bilden. Über die Federsteifigkeit der Membranelemente 8 lässt sich der Querschnitt der Strömungskanäle 11 und damit der Druck im Maskeninnenraum 6 beeinflussen.

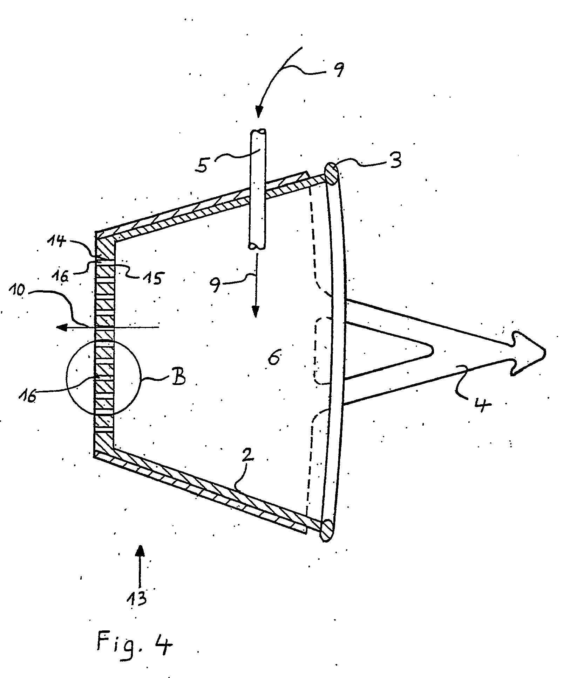

Figur 4 veranschaulicht eine hierin nicht beanspruchte Atemschutzmaske 13, bei der die Ausatemeinrichtung aus einer Vielzahl von durch Membranstreifen 14, 15 begrenzten Strömungskanälen 16 besteht. Die Strömungskanale 16 sind matrixartig über die Vorderseite des Maskenkörpers 2 verteilt angeordnet. Die Membranstreifen 14, 15 sind mit einer elektrischen Potentialquelle verbunden, mit der die Öffnüngsgröße der Strömungskanäle 16 verändert werden kann. Gleiche Komponenten sind mit gleichen Bezugsziffern der Figur 1 versehen.

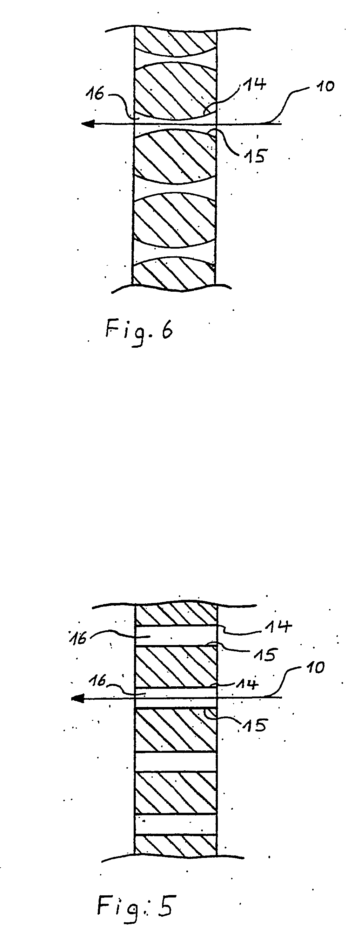

Figur 5 veranschaulicht der besseren Übersicht wegen eine vergrößere Datstellung der Strömungskanäle 16 im Ausschnitt B nach der Figur 4. Gleiche Komponenten sind mit gleichen Bezugsziffer der Figur 4 versehen.

Figur 6 zeigt verengte Strömungskanäle 16 im Ausschnitt B nach der Figur 4, durch eine an die Membranstreifen 14, 15 angeschlossene, in der Figur 6 nicht näher dargestellte Potentialspannungsquelle.

Bei einer alternativen Ausführungsform der hierin nicht beanspruchten 13 sind im Bereich der Ausatemöffnung parallel angeordnete Membranfolien 17 vorgesehen, die mit einzelnen, matrixartig angeordneten Öffnungen 18 versehen sind.

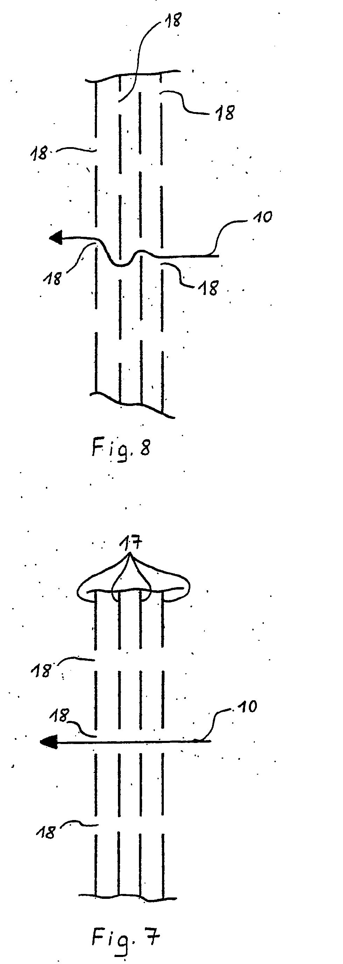

Figur 7 veranschaulicht schematisch die Membranfolien 17 im Ausschnitt B nach der Figur 4. Die Membranfolien 17 sind in der Figur 7 schematisch dargestellt. Sie können auch in Form eines-mehrsthichtigen Gewebes aufgebaut sein.

Die Membranfolien 17 können durch eine nicht näher dargestellte Potentialspannungsquelle in ihrem Abstand zueinander oder in der Länge veränderbar sein, wodurch sich ein Höhenversatz zwischen den Öffnungen 18 ergibt, wie in der Figur 8 veranschaulicht. Der Pfeil 10 zeigt exemplarisch die Durchströmungsrichtung der Membranfolien 17. Ober den Ersatz der Öffnungen 18 zueinander und die Anzahl der Membranfolien 17 ist der Durchströmungswiderstand veränderbar.

## Patentansprüche

1. Atemmaske mit einem Maskenkörper (2) und einer Ausatemeinrichtung, **dadurch gekennzeichnet, dass** die Ausatemeinrichtung aus einer Vielzahl von teilweise überlappend, lamellenartig am Maskenkörper (2) angeordneten, vom Ausatemstrom durchströmbaren Membranelementen (8) besteht.

2. Atemmaske nach Anspruch 1, **dadurch gekennzeichnet, dass** die Membranelemente (8) in Form von einseitig befestigten Biegebalken ausgeführt sind.

3. Atemmaske nach Anspruch 2, **dadurch gekennzeichnet, dass** die Einspannstellen (12) der Membranelemente (8) im Überlappungsbereich der Membranelemente (8) liegen.

4. Atemmaske nach einem der Ansprüche 1 bis 3, **dadurch gekennzeichnet, dass** das Membranmaterial aus einem textilen Werkstoff oder einem Elastomer besteht.

5. Atemmaske nach einem der Ansprüche 1 bis 4, **dadurch gekennzeichnet, dass** das Membranmaterial aus einer Gruppe von Materialien ausgewählt ist, welche infolge elektrischer Felder die Geometrie verändern.

6. Atemmaske nach einem der Ansprüche 1 bis 5, **dadurch gekennzeichnet, dass** das Membranmaterial aus einer Gruppe von Materialien ausgewählt ist, welche infolge elektrischer Felder die Federsteifigkeit verändern.

7. Atemmaske nach Anspruch 5 oder 6, **dadurch gekennzeichnet, dass** das Material eine PVDF-Folie ist.

8. Atemmaske nach einem der Ansprüche 5 bis 7, **dadurch gekennzeichnet, dass** durch elektrische Modulation des Atemgasflusses die Einstellung unterschiedlicher Druckstufen ermöglicht ist.

## Claims

1. Respiratory mask comprising a mask body (2) and an exhalation arrangement, **characterized in that** the exhalation arrangement comprises a plurality of membrane elements which are disposed as partially overlapping lamellas on the mask body and through which expired air can flow.

2. Respiratory mask according to claim 1, **characterized in that** the membrane elements (8) are designed in the form of bendable bars secured at one end.

3. Respiratory mask according to claim 2, **characterized in that** securing positions (12) of the membrane elements (8) are disposed in the overlapping areas of the membrane elements (8).

4. Respiratory mask according to any of the claims 1 to 3, **characterized in that** the membrane material is composed of a textile fabric or an elastomer.

5. Respiratory mask according to any of the claims 1 to 4, **characterized in that** the membrane material is selected from a group of materials which change their geometry in dependence of electrical fields.

6. Respiratory mask according to any of the claims 1 to 5, **characterized in that** the membrane material is selected from a group of materials which change their spring rigidity in dependence of electrical fields.

7. Respiratory mask according to claim 5 or 6, **characterized in that** the material is a PVDF film.

8. Respiratory mask according to any of the claims 5 to 7, **characterized in that** by electrical modulation of the breathing air flow the adjustment of different pressure levels is permitted.

## Revendications

1. Masque respiratoire avec un corps de masque (2) et un dispositif pour l'expiration, **caractérisé en ce que** le dispositif pour l'expiration est constitué par une multitude d'éléments de membrane (8) placés en se chevauchant partiellement à la manière de lamelles sur le corps du masque (2), pouvant être traversés par le flux expiratoire.

2. Masque respiratoire selon la revendication 1, **caractérisé en ce que** les éléments de membrane (8) sont réalisés en forme de barres flexibles fixées d'un côté.

3. Masque respiratoire selon la revendication 2, **caractérisé en ce que** les points d'encastrement (12) des éléments de membrane (8) se trouvent dans la zone de chevauchement des éléments de membrane (8).

4. Masque respiratoire selon l'une des revendications 1 à 3, **caractérisé en ce que** le matériau de la membrane est constitué par un matériau textile ou un élastomère.

5. Masque respiratoire selon l'une des revendications 1 à 4, **caractérisé en ce que** le matériau de la membrane est sélectionné dans un groupe de matériaux qui modifient la géométrie par suite de champs électriques.

6. Masque respiratoire selon l'une des revendications 1 à 5, **caractérisé en ce que** le matériau de la membrane est sélectionné dans un groupe de matériaux qui modifient la flexibilité par suite de champs électriques.

7. Masque respiratoire selon la revendication 5 ou 6, **caractérisé en ce que** le matériau est une feuille de PVDF.

8. Masque respiratoire selon l'une des revendications 5 à 7, **caractérisé en ce que** le réglage de différents étages de pression est rendu possible par modulation électrique du flux de gaz respiratoire.
